# EUROPEAN PATENT APPLICATION

(11) **EP 3 202 327 A1**
(43) Date of publication of application: **09.08.2017**
(21) Application number: 15845793.7
(22) Date of filing: 25.09.2015
(51) Int. Cl.: A61B 8/08

(54) **ULTRASONIC IMAGE PROCESSING DEVICE**

(30) Priority: 01.10.2014 JP 2014203102
(71) Applicant: Hitachi, Ltd., Chiyoda-ku, Tokyo 100-8280 (JP)
(72) Inventor: KOBAYASHI Masaki, Mitaka-shi Tokyo 181-8622 (JP); INOUE Nobuyasu, Mitaka-shi Tokyo 181-8622 (JP); MURASHITA Masaru, Mitaka-shi Tokyo 181-8622 (JP); NAGASE Yuko, Mitaka-shi Tokyo 181-8622 (JP)
(74) Representative: Strehl Schübel-Hopf & Partner
(86) International application number: PCT/JP2015/077180
(87) International publication number: WO 2016/052355

(57) **Abstract**

When measurement of a circumference (abdominal circumference of a fetus) on an ultrasonic image is started, a first mobile marker for designating a major axis start point of an ellipse to be measured is displayed. After the major axis start point is designated, a second mobile marker for designating a major axis end point of the ellipse to be measured and a standard range display graphic are displayed. The standard range display graphic comprises two ellipses in which the major axis start point and second mobile marker constitute a major axis or minor axis and the circumferences are configured to be a lower limit and upper limit of the abdominal circumference. A user can designate the major axis end point while recognizing the positional relationship between the standard range display graphic and the outline of an abdominal cross section. Furthermore, a minor axis end of the ellipse to be measured can be designated using the standard range graphic as a guide.

## Description

### TECHNICAL FIELD

The present disclosure relates to an ultrasound image processor, and in particular to an ultrasound image processor which executes measurement of a circumferential length of an ellipse shape designated on an ultrasound image.

### BACKGROUND

An ultrasound image is formed by an ultrasound diagnostic apparatus which transmits and receives ultrasound to and from a subject. The ultrasound image is used for various examinations and measurements. For example, in order to check a growth state of a fetus, an abdominal circumferential length of the fetus is measured. In this measurement, on an ultrasound image (B-mode image) including a cross section of the abdomen of the fetus, a circumferential length of an outline of an abdominal cross section is measured.

Measurement of the circumferential length on the ultrasound image is generally achieved by a user such as a doctor designating an ellipse shape along an outline of an abdominal cross section of a fetus on an ultrasound image displayed on a display, and calculating the circumferential length of the ellipse shape. For example, the user moves a marker displayed on the ultrasound image using a trackball or the like, and designates ends of the major axis of the abdominal cross section by the marker. Then, the user designates at least one end of the minor axis. With such a process, the circumferential length of the designated ellipse shape is calculated.

There may be cases where a large number of artifacts (noise) exist in the ultrasound image. In such cases, the cross sectional outline becomes unclear on the ultrasound image, and it becomes difficult for the user to accurately designate the ellipse shape. If correct designation of the ellipse shape becomes impossible, a problem of erroneous measurement may result in the measurement of the circumferential length.

A technique for supporting the positioning of a measurement point on the ultrasound image is disclosed. For example, Patent Document 1 discloses that, in a distance measurement of a fetal biparietal diameter, a guide showing a normal range of growth of the fetus is displayed on a screen. The guide includes a line showing a direction passing from the starting point through an end point determining marker, and a line pair including two lines which are orthogonal to the line, which are relatively short, and which show an upper limit and a lower limit of a normal range. The user can use the displayed guide as an estimate, to designate the position of the end point. With such a configuration, erroneous designation of the end point position is prevented.

### CITATION LIST

### PATENT LITERATURE

Patent Document 1: JP 4149015 B

### SUMMARY

### TECHNICAL PROBLEM

Similar to the measurement of distance on the ultrasound image, in the circumferential length measurement for measuring a circumferential length of an ellipse shape by designating the ellipse shape such as the measurement of the abdominal circumferential length, support for the position designation of the ellipse shape is required.

An advantage of the present disclosure lies in supporting a position designation of an ellipse shape in an ultrasound image processor which measures a circumferential length of the ellipse shape designated on an ultrasound image.

### SOLUTION TO PROBLEM

According to one aspect of the present disclosure, there is provided an ultrasound image processor comprising: a measurement unit that measures a circumferential length of an ellipse shape to be measured having a first axis designated on an ultrasound image as one of a major axis and a minor axis, and a second axis which is then designated as the other of the major axis and the minor axis; a marker displaying unit that displays a first axis end point designating marker which moves on the ultrasound image in response to a user operation for designating an end point of the first axis after a starting point of the first axis is designated; and an index figure displaying unit that displays, at least before the end point of the first axis is designated and on the ultrasound image, an index figure having positions of the starting point of the first axis and the first axis end point designating marker as points of reference and showing a standard range of the circumferential range of the ellipse shape to be measured.

According to the above-described configuration, a position of a measurement target ellipse is designated by designation of the major axis and the minor axis. First, a starting point of a first axis which is a major axis or a minor axis is designated. After the starting point of the first axis is designated, an index figure is displayed on the ultrasound image along with a first axis end point designating marker for designating an end point of the first axis. The index figure is a figure having points of reference on positions of the starting point of the first axis and the first axis end point designating marker. For example, the index figure is of an ellipse shape having the positions of the starting point of the first axis and the first axis end point designating marker as ends of the major axis or the minor axis. The user can use the positional relationship between the outline of the measurement target and the index figure on the ultrasound image as an estimate, to designate the position of the first axis end point designating marker. With such a configuration, the user's load upon designating the end point of the first axis can be reduced. Alternatively, precision of the designated position of the end point of the first axis can be improved.

By continuing the display of the index figure even after the designation of the end point of the first axis, it is possible to support, with the index figure, the designation of a starting point of a second axis. The index figure can be represented in various forms, so long as the index figure can show a standard range for the circumferential length of the measurement target.

According to another aspect of the present disclosure, the index figure includes a first two-dimensional figure having an ellipse shape having a direction from the starting point of the first axis through the first axis end point designating marker as one of a major axis direction and a minor axis direction and having, as a circumferential length, a value based on a lower limit value of the standard range, and a second two-dimensional figure having an ellipse shape having a direction from the starting point of the first axis through the first axis end point designating marker as one of a major axis direction and a minor axis direction and having, as a circumferential length, a value based on an upper limit value of the standard range.

According to another aspect of the present disclosure, the marker displaying unit displays, after the end point of the first axis is designated, a second axis end designating marker which moves on the ultrasound image in response to a user operation for designating an end of the second axis, and the index figure displaying unit displays a third two-dimensional figure having an ellipse shape having a line segment connecting the starting point of the first axis and the end point of the first axis as one of a major axis and a minor axis and having the second axis end designating marker as an end of the other of the major axis and the minor axis.

After the end point of the first axis is designated, by displaying, along with the second axis end designating marker, the third two-dimensional figure having an ellipse shape passing through the ends of the first axis and the second axis end point designating marker, it becomes possible for the user to use the third two-dimensional figure as an estimate for designation of the end of the second axis. For example, the end of the second axis can be desirably designated by moving the second axis end designating marker to a position where the third two-dimensional figure and an outline of the measurement target on the ultrasound image coincide with each other, and designating the position as the end of the second axis.

According to another aspect of the present disclosure, the marker displaying unit initially displays the second axis end designating marker on a straight line passing through a middle point of a line segment connecting the starting point of the first axis and the end point of the first axis and perpendicular to the line segment, and in a range between the first two-dimensional figure and the second two-dimensional figure.

### ADVANTAGEOUS EFFECTS OF INVENTION

According to various aspects of the present disclosure, position designation of an ellipse shape can be supported in an ultrasound image processor which measures a circumferential length of an ellipse shape designated on an ultrasound image.

### BRIEF DESCRIPTION OF DRAWINGS

FIG. 1 is a schematic diagram of a structure of an ultrasound diagnostic apparatus according to an embodiment of the present disclosure.
FIG. 2 is a diagram showing a function representing a relationship between a normal range of an abdominal circumferential length and the number of weeks of pregnancy.
FIG. 3 is a diagram showing an example display on a display before a starting point of a major axis of a measurement target ellipse is designated.
FIG. 4 is a diagram showing an example display of a standard range displaying figure.
FIG. 5 shows an example display when a major axis end point designating marker is moved to a candidate position of an end point of the major axis of a measurement target ellipse.
FIG. 6 shows an example display of a minor axis end designating marker and a measurement target ellipse index.
FIG. 7 is a diagram showing another example display of the standard range displaying figure.
FIG. 8 is a diagram showing an example display when a length of a major axis is corrected after a minor axis end of a measurement target ellipse is designated.
FIG. 9 is a diagram showing an example display when a slope of a measurement target ellipse is changed after the measurement target ellipse is designated.
FIG. 10 is a diagram showing a rotational movement of a minor axis end marker.
FIG. 11 is a flowchart showing an example flow of operations of an ultrasound diagnostic apparatus according to an embodiment of the present disclosure.

### DESCRIPTION OF EMBODIMENTS

An embodiment of the present disclosure will now be described with reference to the drawings.

An ultrasound diagnostic apparatus 10 is a medical device which is generally equipped in a medical institution such as a hospital, and which executes an ultrasound diagnosis for a subject. The ultrasound diagnostic apparatus 10 forms an ultrasound image used for various examinations and measurements. The ultrasound diagnostic apparatus 10 has a function to measure a circumferential length of an ellipse shape designated on the ultrasound image which is formed. In the present specification, the term "ellipse shape" not only refers to an ellipse in the mathematical sense, but also includes the concept of a shape slightly deformed from an ellipse (such as an oval shape). In the following, the circumferential length measurement function of the ultrasound diagnostic apparatus 10 will be described while exemplifying measurement of an abdominal circumferential length for checking a growth state of a fetus. In the present specification, the ultrasound diagnostic apparatus 10 is one type of an ultrasound image processor.

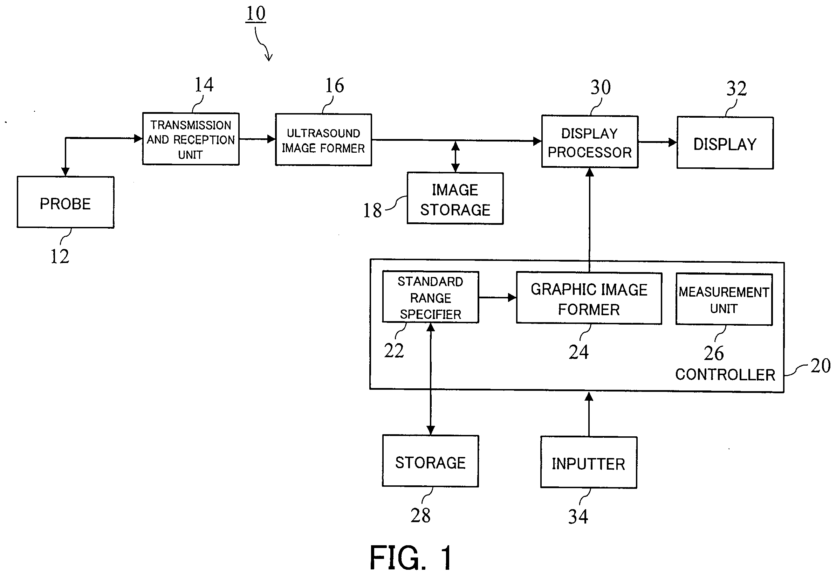

FIG. 1 is a schematic diagram of a structure of the ultrasound diagnostic apparatus 10. A probe 12 is an ultrasound probe which transmits and receives ultrasound to and from a subject. The probe 12 has a transducer array including a plurality of transducers. During transmission, a plurality of transmission signals are supplied from a transmission and reception unit 14 to the plurality of transducers of the transducer array, and the transducers are excited. With this process, an ultrasound beam (transmission beam) is produced. Meanwhile, during reception, when the plurality of transducers of the transducer array receive a reflection echo, in each transducer an acoustic signal is converted into a reception signal, which is an electric signal. A plurality of reception signals thus produced are output to the transmission and reception unit 14. In the present embodiment, a position and an orientation of the probe 12 are adjusted by the user so that the ultrasound is transmitted to and received from an abdomen of the fetus which is the subject.

The transmission and reception unit 14 sends, to the probe 12, a plurality of transmission signals for exciting the plurality of transducers of the probe 12, to cause generation of the ultrasound at the probe 12. The transmission and reception unit 14 further executes phasing addition on the plurality of reception signals produced by the plurality of transducers upon reception of the reflection echo, to form beam data arranged in a scanning direction of the ultrasound beam. Phasing addition is a process to electrically form a reception beam. Each beam data set is formed from a plurality of reflection echo signals arranged in a depth direction. As described, the transmission and reception unit 14 has a function of a transmission beam former and a function of a reception beam former. At the downstream of the transmission and reception unit 14, there is provided a beam data processor including a wave detection circuit or the like, which is not shown in the figures.

An ultrasound image former 16 is formed from, for example, a digital scan converter (DSC), and forms an ultrasound image as a living body image based on the plurality of beam data sets from the transmission and reception unit 14. In the present embodiment, the ultrasound image formed by the ultrasound image former 16 is a B-mode image, and is in particular an image showing a cross section of the abdomen of the fetus. More specifically, the ultrasound image former 16 produces a display frame data array based on a reception frame data array. One reception frame data set is made of a plurality of beam data sets obtained by one beam scanning. The display frame data array forms the B-mode image as a video image. In the present embodiment, a sequence of ultrasound images sequentially produced by the ultrasound image former 16 are sent to a display processor 30 and an image storage 18.

The image storage 18 is a ring buffer which stores a plurality of ultrasound images formed by the ultrasound image former 16. For example, frames from the most recent frame to a predetermined time in the past are temporarily stored in the ring buffer. Alternatively, an image storage may be provided upstream of the ultrasound image former 16. In this case, a group of beam data sets before application of the processing performed by the ultrasound image former 16 are stored for each frame. In the present embodiment, the measurement is not executed in real time during operation, but is executed during an image reproduction operation after freeze. More specifically, a plurality of ultrasound images stored in the image storage 18 are reproduced, and a user selects a particular ultrasound image from the reproduced images. The measurement of the abdominal circumferential length is executed in a state where the ultrasound image is displayed on the screen as a static image.

A controller 20 is formed from, for example, a CPU or a microprocessor, and executes control of each part of the ultrasound diagnostic apparatus 10. The controller 20 has a plurality of functions which operate in the circumferential length measurement, and these functions are shown in FIG. 1 as a plurality of blocks. These functions are realized in the present embodiment as software functions. Alternatively, these functions may be realized using hardware such as, for example, electric/electronic circuits, processors, or the like. Alternatively, these functions may be realized as a cooperation of hardware such as a CPU and a processor, and software (program). The blocks will now be described.

A standard range specifier 22 specifies a lower limit value and an upper limit value of a standard range of the abdominal circumferential length which is the target of measurement. In the present embodiment, the standard range specifier 22 sets a normal range of the abdominal circumferential length calculated from a statistical viewpoint as the standard range. Because the normal range of the abdominal circumferential length differs depending on the number of weeks of pregnancy, the standard range specifier 22 specifies, as the standard range, a lower limit value and an upper limit value of the normal range of the abdominal circumferential length according to the number of weeks of pregnancy. Specifically, the lower limit value and the upper limit value of the standard range of the abdominal circumferential length are specified based on correspondence information showing a correspondence relationship between the lower limit value and the upper limit value of the normal range of the abdominal circumferential length and the number of weeks of pregnancy, and the number of weeks of pregnancy which is input by the user. Alternatively, the lower limit value and the upper limit value of the standard range may be directly input by the user.

A graphic image former 24 forms various graphic elements displayed in an overlapped manner over the ultrasound image in the circumferential length measurement on the ultrasound image. The graphic elements formed by the graphic image former 24 include a first movable marker which moves on the ultrasound image for designating a starting point of a first axis of an ellipse shape to be measured which is a target of the circumferential length measurement, a second movable marker which moves on the ultrasound image for designating an end point of the first axis of the ellipse shape to be measured, a third movable marker which moves on the ultrasound image for designating an end (starting point or end point) of a second axis of the ellipse shape to be measured, a standard range displaying figure which shows a standard range of the abdominal circumferential length specified by the standard range specifier 22, or the like. The graphic elements are not limited to those described above, and an image for supporting the circumferential length measurement, such as a figure showing the designated ellipse shape to be measured, may be formed. The group of formed graphic elements are overlapped over the ultrasound image by the display processor 30 and displayed on a display 32.

The standard range displaying figure showing the standard range of the abdominal circumferential length is displayed on the display 32 after the starting point of the first axis is designated and before the end point of the first axis is designated; that is, during the process of designating the end point of the first axis. By displaying the standard range displaying figure before the end point of the first axis is designated, it becomes possible to use the standard range displaying figure as an estimate for designating the end point of the first axis. In the present embodiment, a figure of an ellipse shape having a line segment connecting the designated starting point position of the first axis and the first movable marker as a major axis or the minor axis is used as the standard range displaying figure.

In addition, the standard range displaying figure continues to be displayed even after the end point of the first axis is designated. With such a configuration, the user can use the standard range displaying figure as an estimate in the positioning of the end of the second axis of the ellipse shape to be measured using the third movable marker.

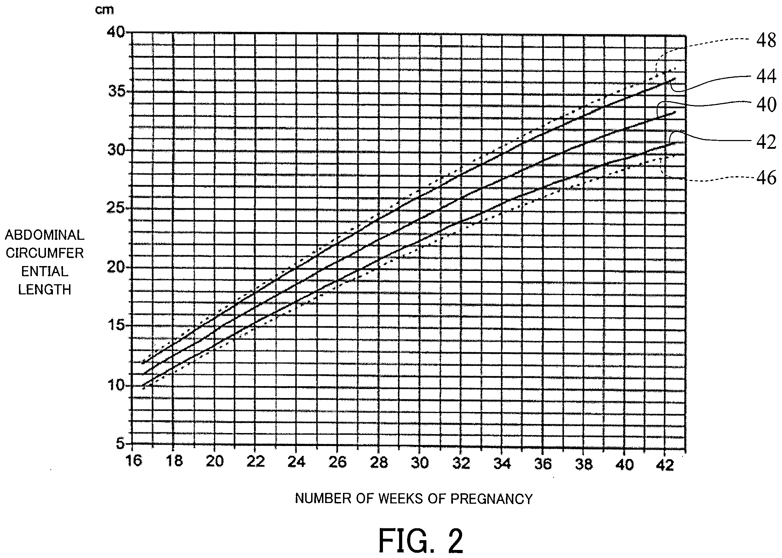

Details of the standard range displaying figure formed by the graphic image former 24 will be described later with reference to FIGs. 2 ∼ 9.

A measurement unit 26 calculates a circumferential length of the ellipse shape to be measured designated on the ultrasound image. The circumferential length of the ellipse shape to be measured is calculated in consideration of the circumferential length of the ellipse shape to be measured on the ultrasound image and the display scale of the ultrasound image.

A storage 28 is, for example, a hard disk drive, a ROM, a RAM, or the like, and stores a program for operating various parts of the ultrasound diagnostic apparatus 10, calculation process results at the ultrasound diagnostic apparatus 10, or the like. The storage 28 also stores the correspondence information showing the correspondence relationship between the lower limit value and the upper limit value of the normal range of the abdominal circumferential length and the number of weeks of pregnancy, which is referred to by the standard range specifier 22. The correspondence information may be stored as a function or in a form of a table or the like.

The display processor 30 executes a process to display, on the display 32, the ultrasound image formed by the ultrasound image former 16. In addition, as described above, the display processor 30 overlaps the group of graphic elements formed by the graphic image former 24 over the ultrasound image and displays the resulting image on the display 32. Further, the display processor 30 may display, on the display 32, a measurement result of the measurement unit 26 in a form or a report or the like.

The display 32 is, for example, a liquid crystal display, and displays the ultrasound image, the group of graphic elements, or the like. An inputter 34 includes a button, a switch, the trackball, or the like, and is used by the user for inputting the number of weeks of pregnancy of the pregnant woman, for moving the first, second, and third movable markers, for designating the ellipse shape to be measured, or the like.

In the following, the details of the standard range displaying figure showing the standard range of the abdominal circumferential length formed by the graphic image former 24 will be described with reference to FIGs. 2 ∼ 9 in connection to FIG. 1.

FIG. 2 is a diagram showing a function showing the relationship between the normal range of the abdominal circumferential length and the number of weeks of pregnancy, stored in the storage 28. Of five graphs shown in FIG. 2, a graph 40 at the center is a graph showing an average value of the abdominal circumferential length in each week of pregnancy. A graph 42 below the graph 40 is a graph showing (the average - 1.5SD (Standard Deviation)), and a graph 44 above the graph 40 is a graph showing (the average + 1.5SD). These graphs are calculated based on a measurement results of the abdominal circumferential length in the past. In each week of pregnancy, about 86% of the entirety of fetuses are included between -1.5SD and +1.5SD. For example, in the 26th week of pregnancy, about 86.6% of the entirety of fetuses have a size of abdominal circumferential length in a range from about 19 cm to about 22 cm. Thus, in the present embodiment, the range of ±1.5SD is set as the standard range of the abdominal circumferential length, with the value at -1.5SD as the lower limit value of the standard range and the value at +1.5SD as the upper limit value of the standard range. The standard range specifier 22 specifies the upper limit value and the lower limit value of the standard range from the function shown in FIG. 2, based on the number of weeks of pregnancy which is input by the user.

A graph 46 positioned further below the graph 42 showing -1.5SD is a graph showing (the average - 2.0SD), and a graph 48 positioned further above the graph 44 showing +1.5SD is a graph showing (the average + 2.0SD). In the present embodiment, the range of ±1.5SD is set as the standard range for the measurement of the abdominal circumferential length, but the standard range is not limited to the range of ±1.5SD. Currently, in Japan, a criterion for the standard range in the measurement of the abdominal circumferential length of the fetus is ±1.5SD, but the criterion for the standard range differs from country to country, and may change even in Japan in the future. In addition, in circumferential length measurements other than the measurement of abdominal circumferential length of the fetus, ranges other than +1.5SD may be set as the standard range. The standard range may be set according to these criteria.

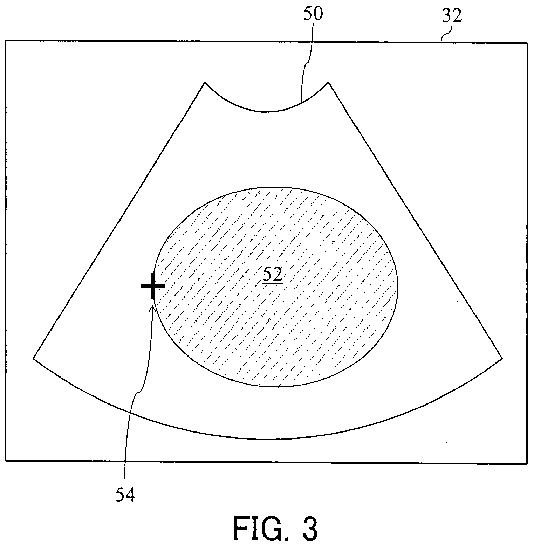

FIG. 3 is a diagram showing an example display of the display 32 when the measurement of the abdominal circumferential length is started. As shown in FIG. 3, on the display 32, a B-mode image 50 formed by the ultrasound image former 16 is displayed. The B-mode image 50 includes a cross section 52 of the abdomen of the fetus. The abdominal cross section 52 is a cross section viewed from the side of the top of the head, and has an ellipse shape.

In the present embodiment, the ellipse shape to be measured is an ellipse, and a major axis and the minor axis of the ellipse are designated by the user. When the measurement of the abdominal circumferential length is started, a first movable marker 54 which moves on the B-mode image 50 for designating a starting point of the first axis of the measurement target ellipse is displayed on the display 32. In the present embodiment, the major axis of the measurement target ellipse is designated as the first axis, and then the minor axis of the measurement target ellipse is designated as a second axis. Alternatively, the minor axis of the measurement target ellipse may be designated first as the first axis.

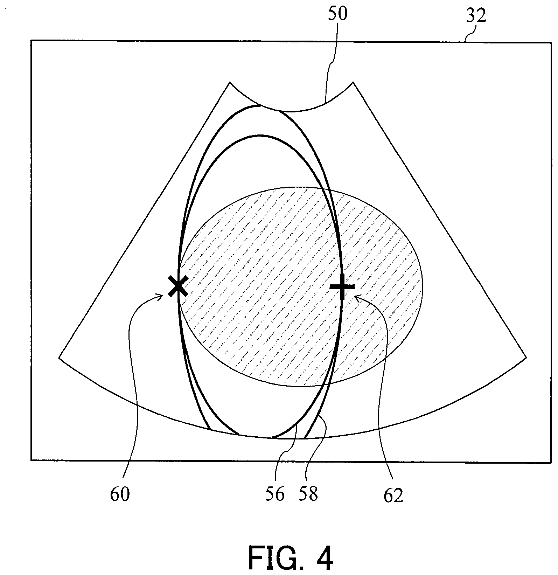

FIG. 4 is a diagram showing an example display of the display 32 in a period between the time when the starting point of the major axis of the measurement target ellipse is designated and the time when the end point of the major axis is designated. When the starting point of the major axis of the measurement target ellipse is designated, the first movable marker is fixed at the designated position of the starting point of the major axis, and a major axis starting point marker 60 is displayed at this position. In the present embodiment, in order to distinguish the first movable marker during movement and the major axis starting point marker 60, these markers are shown with different shapes.

When the user operates the trackball after the major axis starting point of the measurement target ellipse is designated, a second movable marker 62 for designating the end point of the major axis is displayed. In the present embodiment, the shape of the second movable marker 62 is set identical to that of the first movable marker, but alternatively, different shapes may be employed. Along with the second movable marker 62, a lower limit value ellipse 56 and an upper limit value ellipse 58 are displayed which have a line segment connecting the major axis starting point marker 60 and the second movable marker 62 as a major axis or a minor axis. The lower limit value ellipse 56 and the upper limit value ellipse 58 form a standard range displaying figure. A circumferential length of the lower limit value ellipse 56 is a value based on the lower limit value of the standard range specified by the standard range specifier 22. Similarly, a circumferential length of the upper limit value ellipse 58 is a value based on the upper limit value of the standard range specified by the standard range specifier 22. The circumferential lengths of the lower limit value ellipse 56 and the upper limit value ellipse 58 are determined in consideration of the lower limit value and the upper limit value of the specified standard range, and the display scale of the display 32.

As shown in FIG. 4, in the present embodiment, of the lower limit value ellipse 56 and the upper limit value ellipse 58, portions that extend beyond the display range of the B-mode image 50 are not displayed. With such a configuration, it is possible to prevent blockage, by the lower limit value ellipse 56 and the upper limit value ellipse 58, of the display of various pieces of information displayed outside the display range of the B-mode image 50.

In addition, because the circumferential lengths of the lower limit value ellipse 56 and the upper limit value ellipse 58 are constants, when the distance between the second movable marker 62 and the major axis starting point marker 60 is close, the lower limit value ellipse 56 and the upper limit value ellipse 58 assume shapes elongated in the longitudinal direction. Such a shape cannot be used as a support for positioning the major axis end point, and also complicates the display. Therefore, a configuration may be employed in which, when the distance between the second movable marker 62 and the major axis starting point marker 60 is less than or equal to a predetermined value, the lower limit value ellipse 56 and the upper limit value ellipse 58 are not displayed.

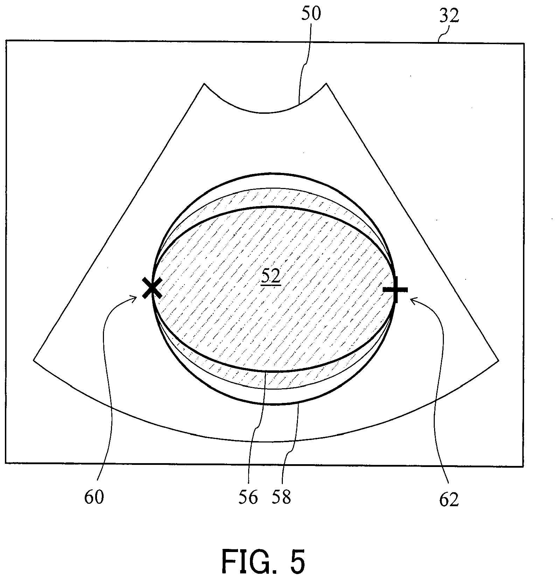

FIG. 5 shows an example display when the second movable marker 62 is moved to a candidate position of the end point of the major axis of the measurement target ellipse. As can be understood from comparison between FIGs. 4 and 5, because the circumferential lengths of the lower limit value ellipse 56 and the upper limit value ellipse 58 are held constant, as the second movable marker 62 is moved to the right, the widths, in the up-and-down direction, of the lower limit value ellipse 56 and the upper limit value ellipse 58 are reduced, and the shapes change to extend toward the right side. In a state where the second movable marker 62 is moved to the major axis end point candidate position, the outline of the abdominal cross section 52 is compared with the positional relationship between the lower limit value ellipse 56 and the upper limit value ellipse 58, so as to enable the user to judge whether or not the current position of the second movable marker 62 is at an appropriate position as the major axis end point position of the measurement target ellipse.

As shown in FIG. 5, when the outline of the abdominal cross section 52 is located in a region between the lower limit value ellipse 56 and the upper limit value ellipse 58 (a region inside the upper limit value ellipse 58 and outside the lower limit value ellipse 56), it is possible to comprehend that the abdominal circumferential length of the fetus which is the subject falls within the standard range before the major axis end point position of the measurement target ellipse is designated. That is, this means that the current position of the second movable marker 62 is at an appropriate position as the end point of the major axis of the measurement target ellipse, and that the major axis starting position of the measurement target ellipse is also designated at an appropriate position.

In contrast, if the outline of the abdominal cross section 52 is not located in a region between the lower limit value ellipse 56 and the upper limit value ellipse 58, various possibilities may be considered. First, a case may be considered in which the current position of the second movable marker 62 is not at an appropriate position as the major axis end point of the measurement target ellipse. Secondly, a case may be considered in which the major axis starting point of the measurement target ellipse which is already designated is not at an appropriate position. Thirdly, a case may be considered in which the abdominal cross section 52 included in the B-mode image 50 is not an appropriate cross section. Fourthly, a case may be considered in which the size of the fetus actually falls outside the standard range. When the size of the fetus is not normal, it is typical to again capture the ultrasound image and again measure the size. In other words, in any of the above-described cases, superior measurement result cannot be obtained when the current position of the second movable marker 62 is set as the major axis end point of the measurement target ellipse. Therefore, when the outline of the abdominal cross section 52 is not located in a region between the lower limit value ellipse 56 and the upper limit value ellipse 58, the user can comprehend that the current position of the second movable marker 62 is not at an appropriate position as the major axis end point of the measurement target ellipse.

In this manner, according to the present embodiment, the user can comprehend whether or not the end point candidate position is at an appropriate position as the major axis end point of the measurement target ellipse, before the major axis end point of the measurement target ellipse is designated. When the candidate position is not appropriate as the major axis end point of the measurement target ellipse, the user can comprehend it at an early stage, and can execute countermeasures such as review of the major axis end point position or the major axis starting point position of the measurement target ellipse, and re-selection or re-capturing of the ultrasound image at an early stage. Thus, it is possible to reduce the amount of re-doing work by the user, and to reduce the work effort related to the circumferential length measurement.

In addition, also in a case where the outline of the abdominal cross section 52 is unclear near the major axis end point of the measurement target ellipse, the appropriate major axis end point position can be deduced based on the positional relationship between the outlines of other portions (for example, outline near the minor axis end) and the standard range displaying figure.

In the present embodiment, the lower limit value ellipse 56 and the upper limit value ellipse 58 are displayed as the standard range displaying figure. Alternatively, various configurations may be employed for the standard range displaying figure, so long as the standard range of the abdominal circumferential length can be shown. For example, the lower limit value ellipse 56 and the upper limit value ellipse 58 may be displayed with broken lines in order for the user to more easily understand the outline of the abdominal cross section 52, or a portion between the lower limit value ellipse 56 and the upper limit value ellipse 58 may be colored in order for the user to more easily understand the standard range.

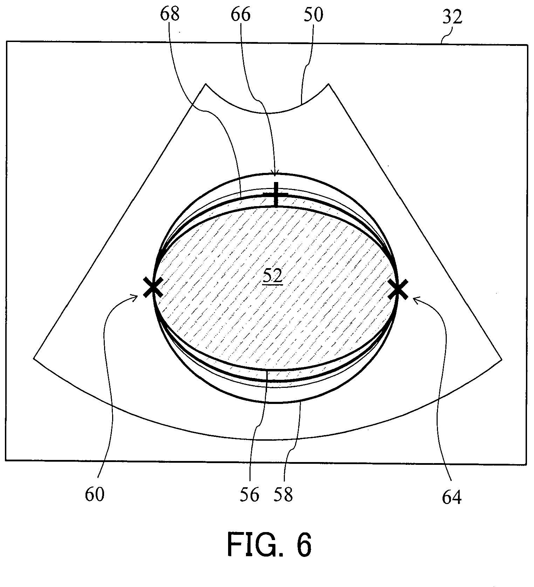

FIG. 6 is a diagram showing an example display after the major axis end point of the measurement target ellipse is designated. When the major axis end point of the measurement target ellipse is designated by the user pressing a determination button or the like, the second movable marker is fixed at the designated end point position, and a major axis end point marker 64 is displayed at this position. When the major axis end point is designated, a third movable marker 66 for designating an end of the minor axis of the measurement target ellipse (starting point or end point) is displayed. Because the third movable marker 66 is for designating an end of the minor axis of the measurement target ellipse, the marker passes through a midpoint of the major axis of the measurement target ellipse (line segment connecting the major axis starting point marker 60 and the major axis end point marker 64), and moves in a direction perpendicular to the major axis. The lower limit value ellipse 56 and the upper limit value ellipse 58 continue to be displayed even after the designation of the major axis end point.

An initial position for displaying the third movable marker 66 for designating the minor axis end of the measurement target ellipse may be determined based on the standard range displaying figure. The position designated as the minor axis end of the measurement target ellipse may be considered to be between the lower limit value ellipse 56 and the upper limit value ellipse 58. Therefore, for example, the initial display position of the third movable marker 66 may be set at a point on a straight line perpendicular to the line segment connecting the major axis starting point marker 60 and the major axis end point marker 64 and passing through the midpoint of the line segment, and positioned between the lower limit value ellipse 56 and the upper limit value ellipse 58. Desirably, an intermediate point between the lower limit value ellipse 56 and the upper limit value ellipse 58 or a nearby point is set as the initial display position of the third movable marker 66. With such a configuration, a movement distance of the third movable marker 66 for designating the minor axis end of the measurement target ellipse can be reduced.

Along with the third movable marker 66, there is displayed a measurement target ellipse index 68 having the line segment connecting the major axis starting point marker 60 and the major axis end point marker 64 as the major axis and the third movable marker 66 as an end of the minor axis. The measurement target ellipse index 68 is a figure which shows the shape of the measurement target ellipse. When the third movable marker 66 is moved in the up-and-down direction, the shape of the measurement target ellipse index 68 is also changed to extend in the up-and-down direction.

The user can use the measurement target ellipse index 68 as an estimate when designating the minor axis end of the measurement target ellipse. For example, the user can move the third movable marker 66 to a position where the outlines of the abdominal cross section 52 and the measurement target ellipse index 68 overlap each other, and designate the position as the minor axis end of the measurement target ellipse, to thereby accurately designate the position of the minor axis end. With regard to the minor axis end of the measurement target ellipse, when the position of one of the ends is designated, the position of the other end is automatically determined, and thus, only one of the minor axis ends is designated. When the minor axis end of the measurement target ellipse is designated, the measurement target ellipse is determined. The measurement unit 26 then calculates the circumferential length of the determined measurement target ellipse.

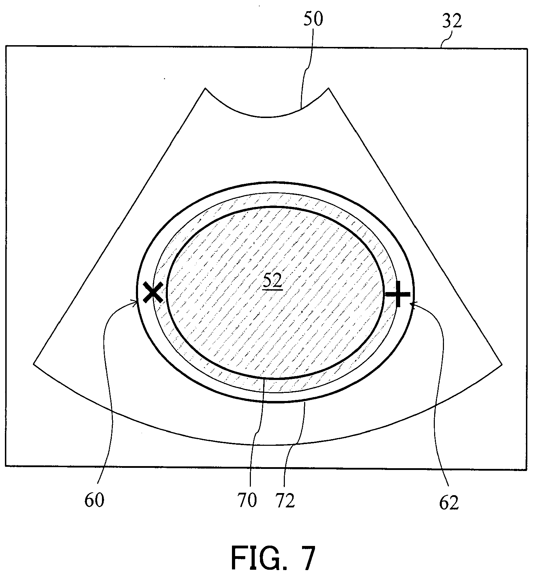

FIG. 7 is a diagram showing an alternative example of the standard range displaying figure. The lower limit value ellipse 56 and the upper limit value ellipse 58 shown in FIGs. 4 - 6 are set as ellipses passing through the major axis starting point marker 60 and the second movable marker 62 (or the major axis end point marker 64). In the alternative configuration of FIG. 7, a lower limit value ellipse 70 and an upper limit value ellipse 72 are set as ellipses which do not pass through the major axis starting point marker 60 and the second movable marker 62 (or the end point marker), and instead, pass through nearby points of these markers.

Specifically, an ellipse in which an ellipse having the line segment connecting the major axis starting point marker 60 and the second movable marker 62 as the major axis or the minor axis (hereinafter referred to as a "basic ellipse") is reduced in similitude (the circumferential length of which is the lower limit value of the standard range) is set as the lower limit value ellipse 70, and an ellipse in which the basic ellipse is enlarged in similitude (the circumferential length of which is the upper limit value of the standard range) is set as the upper limit value ellipse 72. Alternatively, an ellipse in which the major axis and the minor axis of the basic ellipse are shortened by the same length may be set as the lower limit value ellipse 70, and an ellipse in which the major axis and the minor axis of the basic ellipse are lengthened by the same length may be set as the upper limit value ellipse 72.

As shown in FIG. 7, by displaying the lower limit value ellipse 70 and the upper limit value ellipse 72 passing through points slightly deviated from the major axis starting point marker 60 and the second movable marker 62, it becomes possible to determine the end point of the major axis of the measurement target ellipse to include, in a region between the lower limit value ellipse 70 and the upper limit value ellipse 72, the entirety of the outline of the abdominal cross section 52 including regions near the starting point and the end point of the major axis of the measurement target ellipse.

In the example configuration of FIG. 7, the lower limit value ellipse 70 and the upper limit value ellipse 72 are set as ellipses which pass through points slightly deviated from the major axis starting point marker 60 and the second movable marker 62. Alternatively, a configuration may be employed in which an ellipse having a line segment connecting a point which is deviated by a predetermined value from the position of the second movable marker 62 toward the side of the major axis starting point marker 60 and the major axis starting point marker 60 as the major axis or the minor axis is set as the lower limit value ellipse, and an ellipse in which a line segment connecting a point which is deviated by a predetermined value from the position of the second movable marker 62 toward a side opposite from the major axis starting point marker 60 and the major axis starting point marker 60 as the major axis or the minor axis is set as the upper limit value ellipse. With such a configuration, the lower limit value ellipse 70 and the upper limit value ellipse 72 are ellipses which pass through the major axis starting point marker 60 and a region near the second movable marker 62. In this case, the end point of the major axis of the measurement target ellipse can be determined to include, in a region between the lower limit value ellipse 70 and the upper limit value ellipse 72, the outline of the abdominal cross section 52 including a region near the end point of the major axis of the measurement target ellipse.

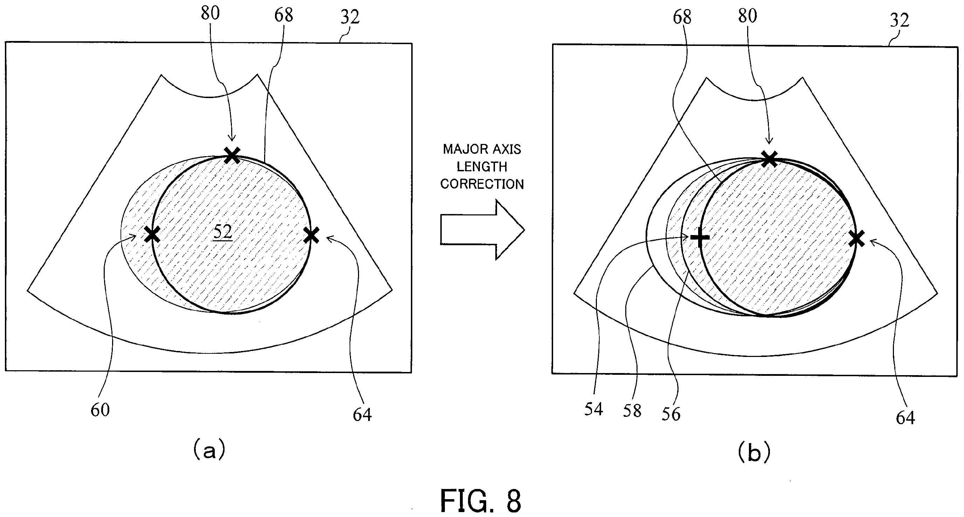

FIG. 8 is a diagram showing an example display when the length of the major axis of the measurement target ellipse is corrected after the minor axis end of the measurement target ellipse is designated. The ultrasound diagnostic apparatus 10 has a function to correct the starting point and the end point of the major axis of the measurement target ellipse which are designated, or the minor axis end. In the following, there will be described an example display in the case where the starting point of the major axis is corrected after the minor axis end of the measurement target ellipse is designated. When the length of the minor axis is to be corrected, referring again to FIG. 6, the user again designates the minor axis end with the third movable marker 66.

As shown in (A) of FIG. 8, when the minor axis end of the measurement target ellipse is designated, a minor axis end marker 80 is displayed at a position of the minor axis end. The measurement target ellipse index 68 in which the position and the size are determined by the major axis starting point marker 60, the major axis end point marker 64, and the minor axis end marker 80 continues to be displayed. Further, although not shown in (B) of FIG. 8, the lower limit value ellipse 56 and the upper limit value ellipse 58 continue to be displayed. Then, when the user notices that the position of the major axis starting point marker 60 is deviated from the outline position of the abdominal cross section 52 and enters a major axis length correction mode by pressing a correction button included in the inputter 34, as shown in (B) of FIG. 8, the major axis starting point marker 60 is deleted, and the first movable marker 54 is again displayed at or near the position where the major axis starting point marker 60 was displayed.

In the major axis correction mode also, the lower limit value ellipse 56 and the upper limit value ellipse 58 are displayed. In this case, the displayed lower limit value ellipse 56 is an ellipse having a length of the minor axis (or the major axis) identical to the length of the minor axis of the designated measurement target ellipse index 68, having the major axis end point marker 64 as an end of the major axis (or the minor axis), and having the circumferential length identical to the lower limit value of the standard range. Similarly, the upper limit value ellipse 58 is an ellipse having a length of the minor axis (or the major axis) identical to the length of the minor axis of the designated measurement target ellipse index 68, having the major axis end point marker 64 as an end of the major axis (or the minor axis), and having the circumferential length identical to the upper limit value of the standard range.

As described with reference to FIG. 5, in the designation of the position of the major axis end point, the positional relationship between the outline of the abdominal cross section 52 (in particular, the outline near the minor axis) and the lower limit value ellipse 56 and the upper limit value ellipse 58 is used as the estimate for the position designation. In the major axis correction mode, based on a presumption that the setting of the minor axis of the measurement target ellipse index 68 is appropriate, the lower limit value ellipse 56 and the upper limit value ellipse 58 directly show a range which can be considered to be appropriate as the major axis starting point (that is, a range of the major axis starting point where the circumferential length of the measurement target ellipse is within the standard range). The user can correct the major axis starting point position using the lower limit value ellipse 56 and the upper limit value ellipse 58 as an estimate.

In order to correct the length of the major axis, the first movable marker 54 is moved in a direction of the major axis of the measurement target ellipse. In the example of (B) of FIG. 8, the first movable marker 54 is moved toward the left direction to lengthen the length of the major axis. In addition, with the movement of the first movable marker 54 toward the left direction, the shape of the measurement target ellipse index 68, which continues to be displayed from the time before the designation of the minor axis end, is changed to extend in the left direction. The user can further use the measurement target ellipse index 68 as the estimate for the designation of the corrected starting point position. With the change of the shape of the measurement target ellipse 68 to extend in the left direction, the position of the minor axis end marker 80 which shows the position of the minor axis end of the measurement target ellipse index 68 is also moved. More specifically, for example, when the first movable marker 54 is moved to the left by 2 cm, the minor axis end marker 80 is moved to the left by a half of the distance, 1 cm. In this manner, the minor axis end marker 80 continues to show the end of the minor axis (or the major axis) of the measurement target ellipse index 68.

In FIG. 8, a case is described where the major axis starting point of the measurement target ellipse is corrected. In a case where the major axis end point is corrected, a similar display as above is shown. In this case, the major axis end point marker 64 is deleted, and the second movable marker is displayed. The lower limit value ellipse 56 and the upper limit value ellipse 58 are displayed while having the minor axes (or the major axes) with identical lengths as the length of the minor axis of the measurement target ellipse index 68, having the major axis starting point marker 60 as one end of the major axis (or the minor axis), and having the circumferential lengths of the lower limit value and the upper limit value of the standard range, respectively.

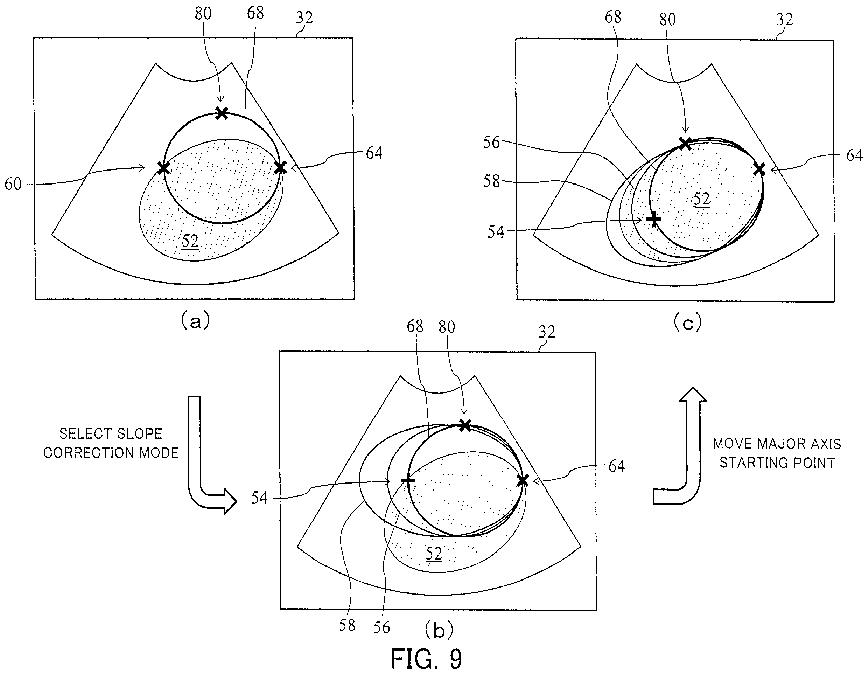

The ultrasound diagnostic apparatus 10 can also correct the slope of the measurement target ellipse. FIG. 9 is a diagram showing an example display when the slope of the measurement target ellipse is corrected after the measurement target ellipse is designated. Although not shown in (A) of FIG. 9 also, the lower limit value ellipse 56 and the upper limit value ellipse 58 continue to be displayed. In this case, similar to the example configuration of FIG. 8, the user presses the correction button in the display state shown in (A) of FIG. 9 in which the minor axis end of the measurement target ellipse is designated, to enter a slope correction mode. Similar to the above, as shown in (B) of FIG. 9, the major axis starting point marker 60 is deleted and the first movable marker 54 is again displayed.

In the state of (B) of FIG. 9, similar to (B) of FIG. 8, the lower limit value ellipse 56 and the upper limit value ellipse 58 are displayed while having the minor axes (or the major axes) of the same length as the minor axis of the measurement target ellipse index 68, having the major axis end point marker 64 as an end of the major axis (or the minor axis), and having the circumferential lengths of the lower limit value and the upper limit value of the standard range, respectively. The user corrects the slope of the measurement target ellipse index 68 using the lower limit value ellipse 56 and the upper limit value ellipse 58 as an estimate.

The first movable marker 54 is set movable in a rotational direction centered at the position of the major axis end point (position of the major axis end point marker 64) of the measurement target ellipse. With this configuration, the slope of the measurement target ellipse is corrected. As shown in (C) of FIG. 9, when the first movable marker 54 is rotated around the major axis end point marker 64, the lower limit value ellipse 56 and the upper limit value ellipse 58 are rotated around the major axis end point marker 64, following the rotational movement of the fist movable marker 54. With such a configuration, it is possible for the user to use the lower limit value ellipse 56 and the upper limit value ellipse 58 as an estimate for designating the corrected slope of the measurement target ellipse. In other words, the measurement target ellipse index 68 is rotated so that the outline of the abdominal cross section 52 lies within the region between the lower limit value ellipse 56 and the upper limit value ellipse 58.

With the rotational movement of the first movable marker 54, the measurement target ellipse index 68 which continues to be displayed from the time before the designation of the minor axis end is also rotated following the first movable marker 54. With such a configuration, the user can further use the measurement target ellipse index 68 in the designation of the slope of the measurement target ellipse. With the rotational movement of the measurement target ellipse index 68, the position of the minor axis end marker 80 showing the position of the minor axis end of the measurement target ellipse index 68 is also rotated.

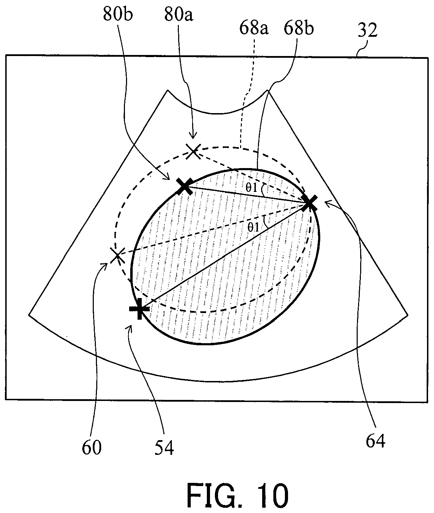

FIG. 10 is a diagram showing the rotational movement of the minor axis end marker 80. In the example configuration of FIG. 10, the first movable marker is rotated around the major axis end point marker 64 from the position 60 to the position 54. If the rotational angle in this case is θ1, the minor axis end marker is also rotated by the angle θ1 around the major axis end point marker 64 from a position 80a to a position 80b. With this process, the minor axis end marker 80 continues to show the end of the minor axis (or the major axis) of the measurement target ellipse index 68.

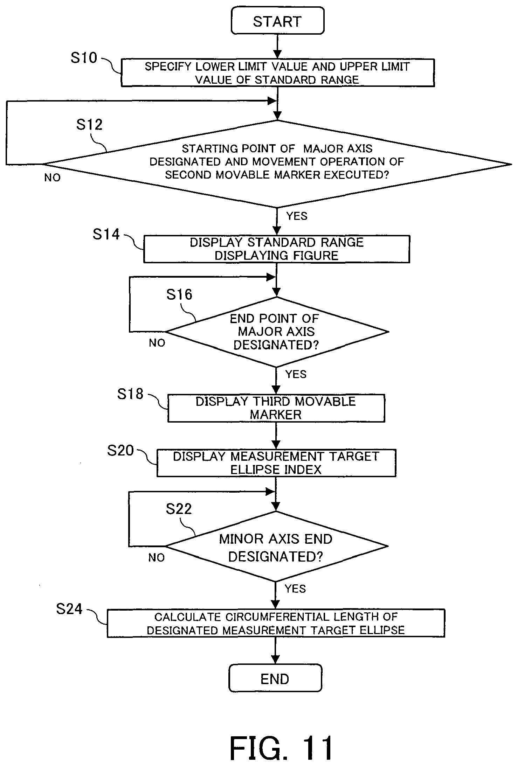

FIG. 11 is a flowchart showing an example flow of operations of the ultrasound diagnostic apparatus 10. The steps shown in FIG. 11 will now be described with reference to FIG. 1.

When the user designates an ultrasound image to be used for circumferential length measurement, and the circumferential length measurement is started at the ultrasound diagnostic apparatus 10, in step S10, the standard range specifier 22 specifies the lower limit value and the upper limit value of the standard range of the abdominal circumferential length of a fetus based on the number of weeks of pregnancy of the pregnant woman which is input by the user.

In step S12, the controller 20 judges whether or not the starting point of the major axis of the measurement target ellipse is designated by the user operating the first movable marker, and whether or not the movement operation of the second movable marker for designating the end point of the major axis of the measurement target ellipse is executed by the user operating the trackball or the like after designating the starting point.

In step S 14, the display processor 30 displays the major axis starting point marker at the starting point position of the major axis of the measurement target ellipse. Further, the display processor 30 displays the second movable marker on the ultrasound image, and displays, as the standard range displaying figures, a lower limit value ellipse having the line segment connecting the major axis starting point marker and the second movable marker as the major axis or the minor axis and the lower limit value of the standard range specified in step S10 as the circumferential length, and the upper limit value ellipse having the line segment connecting the major axis starting point marker and the second movable marker as the major axis or the minor axis and the upper limit value of the standard range specified in step S10 as the circumferential length.

In step S16, the controller 20 judges whether or not the end point of the major axis of the measurement target ellipse is designated by the user. Until the end point of the major axis is designated, the display processor 30 continues to display the second movable marker and the standard range displaying figure (the lower limit value ellipse and the upper limit value ellipse).

When the user designates the end point of the major axis of the measurement target ellipse, in step S18, the display processor 30 displays the major axis end point marker at the position of the major axis end point. In addition, the display processor 30 displays the third movable marker for designating the minor axis end of the measurement target ellipse. Further, in step S20, the display processor 30 displays the measurement target ellipse index showing the shape of the measurement target ellipse. The measurement target ellipse index is an ellipse having a line segment connecting the major axis starting point marker and the major axis end point marker as the major axis, and the third movable marker as the minor axis end. Further, the display of the lower limit value ellipse and the upper limit value ellipse is continued even after the end point of the major axis is designated.

In step S22, the controller 20 judges whether or not the minor axis end of the measurement target ellipse is designated by the user. Until the minor axis end is designated, the display processor 30 continues to display the third movable marker, the lower limit value ellipse, the upper limit value ellipse, and the measurement target ellipse index.

In step S24, the measurement unit 26 measures the circumferential length of the designated measurement target ellipse.

The present embodiment has been described exemplifying the measurement of the abdominal circumferential length of a fetus. The present disclosure, however, is not limited to such a configuration, and may be applied to any measurement of the circumferential length of an ellipse shape designated on an ultrasound image. In this case, as the table or function showing the lower limit value and the upper limit value of the standard range stored in the storage 28, a table or function corresponding to the measurement target is prepared.

In addition, in the present embodiment, the ultrasound diagnostic apparatus 10 is exemplified as an ultrasound image processor. Alternatively, for example, a PC or the like may be used as the ultrasound image processor. In this case, the ultrasound image formed by the ultrasound diagnostic apparatus is sent to the PC, and the PC executes the specification of the standard range, overlapping of the graphic image, measurement of the circumferential length, or the like.

### REFERENCE SIGNS LIST

10 ULTRASOUND DIAGNOSTIC APPARATUS; 12 PROBE; 14 TRANSMISSION AND RECEPTION UNIT; 16 ULTRASOUND IMAGE FORMER; 18 IMAGE STORAGE; 20 CONTROLLER; 22 STANDARD RANGE SPECIFIER; 24 GRAPHIC IMAGE FORMER; 26 MEASUREMENT UNIT; 28 STORAGE; 30 DISPLAY PROCESSOR; 32 DISPLAY; 34 INPUTTER.

## Claims

1. An ultrasound image processor comprising:
a measurement unit that measures a circumferential length of an ellipse shape to be measured having a first axis designated on an ultrasound image as one of a major axis and a minor axis, and a second axis which is then designated as the other of the major axis and the minor axis;
a marker displaying unit that displays a first axis end point designating marker which moves on the ultrasound image in response to a user operation for designating an end point of the first axis after a starting point of the first axis is designated; and
an index figure displaying unit that displays, at least before the end point of the first axis is designated and on the ultrasound image, an index figure having positions of the starting point of the first axis and the first axis end point designating marker as points of reference and showing a standard range of the circumferential length of the ellipse shape to be measured.

2. The ultrasound image processor according to Claim 1, wherein
the index figure includes a first two-dimensional figure having an ellipse shape having a direction from the starting point of the first axis through the first axis end point designating marker as one of a major axis direction and a minor axis direction and having, as a circumferential length, a value based on a lower limit value of the standard range, and a second two-dimensional figure having an ellipse shape having a direction from the starting point of the first axis through the first axis end point designating marker as one of a major axis direction and a minor axis direction and having, as a circumferential length, a value based on an upper limit value of the standard range.

3. The ultrasound image processor according to Claim 2, wherein
the marker displaying unit displays, after the end point of the first axis is designated, a second axis end designating marker which moves on the ultrasound image in response to a user operation for designating an end of the second axis, and
the index figure displaying unit displays a third two-dimensional figure having an ellipse shape having a line segment connecting the starting point of the first axis and the end point of the first axis as one of a major axis and a minor axis and having the second axis end designating marker as an end of the other of the major axis and the minor axis.

4. The ultrasound image processor according to Claim 3, wherein
the marker displaying unit initially displays the second axis end designating marker on a straight line passing through a midpoint of a line segment connecting the starting point of the first axis and the end point of the first axis and perpendicular to the line segment, and in a range between the first two-dimensional figure and the second two-dimensional figure.
